# EUROPEAN PATENT APPLICATION

(11) **EP 4 491 608 A1**
(43) Date of publication of application: **15.01.2025**
(21) Application number: 23860782.4
(22) Date of filing: 24.08.2023
(51) Int. Cl.: C07C 51/43, C07C 57/04, C07C 51/25

(54) **METHOD FOR PREPARING HIGH-PURITY (METH)ACRYLIC ACID**

(30) Priority: 30.08.2022 KR 20220109483; 16.08.2023 KR 20230107030
(71) Applicant: LG Chem, Ltd., Yeongdeungpo-gu Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2023/012560
(87) International publication number: WO 2024/049105

(57) **Abstract**

Provided is a method for preparation of (meth)acrylic acid including: bringing a mixed gas including (meth)acrylic acid into contact with water in an absorption column to obtain a (meth)acrylic acid aqueous solution; supplying the (meth)acrylic acid aqueous solution to a crystallizer and performing crystallization to obtain purified (meth)acrylic acid and a mother liquor separated from the purified (meth)acrylic acid; supplying a part of the mother liquor to an extraction column and supplying the rest to a water separation column; bringing the mother liquor into contact with an extraction solvent in the extraction column and then supplying an upper discharge stream of the extraction column to the water separation column; performing separation in the water separation column into an upper discharge stream of the water separation column including water and a lower discharge stream of the water separation column including (meth)acrylic acid and high-boiling point by-products; and supplying the lower discharge stream of the water separation column to a high-boiling point by-product separation column and supplying an upper discharge stream of the high-boiling point by-product separation column including (meth)acrylic acid to the crystallizer.

## Description

### [Technical Field]

### CROSS-REFERENCE TO RELATED APPLICATIONS

The present application claims the benefit of priorities to Korean Patent Application No. 10-2022-0109483, filed on August 30, 2022, and Korean Patent Application No. 10-2023-0107030, filed on August 16, 2023, the entire contents of which are incorporated herein as a part of the specification.

### Technical Field

The present invention relates to a method for preparation of high-purity (meth)acrylic acid.

### [Background Art]

(Meth)acrylic acid is generally prepared by a method of subjecting a compound such as propane, propylene, and (meth)acrolein to a gas phase oxidation reaction in the presence of a catalyst. For example, propane, propylene, and the like are converted through (meth)acrolein into (meth)acrylic acid by a gas phase oxidation reaction in the presence of an appropriate catalyst in a reactor, and a mixed gas including (meth)acrylic acid, unreacted propane or propylene, (meth)acrolein, inert gas, carbon dioxide, water vapor, and various organic by-product by the reaction (such as acetic acid, low-boiling point by-products, and high-boiling point by-products) is obtained in a latter stage of the reactor.

The (meth)acrylic acid-containing mixed gas is brought into contact with an absorption solvent such as water in an absorption column to be recovered as a (meth)acrylic acid aqueous solution. Further, as a subsequent process for recovering the (meth)acrylic acid included in the (meth)acrylic acid aqueous solution, it is common to involve processes such as extraction, distillation, and purification. In order to improve recovery efficiency of the (meth)acrylic acid, various methods of adjusting process conditions, process order, or the like have been suggested.

However, since the absorption solvent such as water used in the absorption column has a high specific heat, significantly high energy usage is demanded in separating by-products from the (meth)acrylic acid aqueous solution including water through a process such as distillation. Meanwhile, when the subsequent process is simplified and shortened for reducing the energy usage, the energy usage may be decreased, but it is difficult to obtain high-purity (meth)acrylic acid.

Furthermore, acetic acid and high-boiling point by-products which are main by-products of a preparation process of (meth)acrylic acid are separated and removed so that they do not accumulate in the system, and in this process, loss of (meth)acrylic acid, which is the desired product, is incurred.

Therefore, it is urgent to introduce a technology which may reduce the energy usage throughout the entire process, while also minimizing loss of high-purity (meth)acrylic acid from a (meth)acrylic acid aqueous solution.

### [Disclosure]

### [Technical Problem]

In order to solve the problems mentioned in the Background Art, an object of the present invention is to provide a method for preparation of (meth)acrylic acid, which may further reduce energy usage in a purification process, while securing a high (meth)acrylic acid recovery rate.

### [Technical Solution]

In one general aspect, a method for preparation of (meth)acrylic acid includes: bringing a mixed gas including (meth)acrylic acid into contact with water in an absorption column to obtain a (meth)acrylic acid aqueous solution; supplying the (meth)acrylic acid aqueous solution to a crystallizer and performing crystallization to obtain purified (meth)acrylic acid and a mother liquor separated from the purified (meth)acrylic acid; supplying a part of the mother liquor to an extraction column and supplying the rest to a water separation column; bringing the mother liquor into contact with an extraction solvent in the extraction column and then supplying an upper discharge stream of the extraction column to the water separation column; performing separation in the water separation column into an upper discharge stream of the water separation column including water and a lower discharge stream of the water separation column including (meth)acrylic acid and high-boiling point by-products; and supplying the lower discharge stream of the water separation column to a high-boiling point by-product separation column and supplying an upper discharge stream of the high-boiling point by-product separation column including (meth)acrylic acid to the crystallizer.

### [Advantageous Effects]

According to the method for preparation of (meth)acrylic acid according to the present invention, an amount of water in the system may be minimized in a process after an absorption column, a (meth)acrylic acid aqueous solution discharged from the absorption column may be supplied to a crystallizer to obtain high-purity (meth)acrylic acid, distillation is performed using extraction of a crystallized mother liquor in a subsequent process to reduce energy usage, and high-boiling point by-products may be efficiently removed to decrease (meth)acrylic acid loss.

In addition, an extraction column, a water separation column, and a layer separator may be provided after the absorption column to separate and remove acetic acid as a by-product, and thus, the loss of (meth)acrylic acid from an upper portion of the absorption column may be decreased as compared with the case in which all acetic acid is discharged from the upper portion of the absorption column.

Furthermore, the high-boiling point by-products may be separated after the water separation column to prevent accumulation of the high-boiling point by-products in the system, thereby obtaining high-purity purified (meth)acrylic acid, and an upper discharge stream of the high-boiling point by-products are supplied again to the crystallization, thereby further decreasing the loss of (meth)acrylic acid.

### [Description of Drawings]

FIG. 1 is a process flow chart showing a method for preparation of (meth)acrylic acid according to an exemplary embodiment of the present invention.
FIGS. 2 and 3 are process flow charts showing a method for preparation of (meth)acrylic acid according to the comparative examples of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a fluid flow in a process, or may refer to a fluid itself flowing in a pipe. Specifically, the stream may refer to both a fluid itself flowing in a pipe connecting each device and a fluid flow. In addition, the fluid may refer to a gas or liquid, and a case in which a solid substance is included in the fluid is not excluded.

Meanwhile, in the devices such as an absorption column, a degassing column, a distillation column or a distillation column, and a crystallizer, unless otherwise particularly stated, a "lower portion" of the device refers to a point at a height of 95% to 100% from top to bottom of the device, specifically a lowest part (bottom). Likewise, unless otherwise particularly stated, an "upper portion" of the device refers to a point at a height of 0% to 5% from top to bottom of the device, specifically a highest part (top).

Hereinafter, the present invention will be described in more detail for better understanding of the present invention.

A method for preparation of (meth)acrylic acid according to an exemplary embodiment of the present invention may include: bringing a mixed gas including (meth)acrylic acid into contact with water in an absorption column to obtain a (meth)acrylic acid aqueous solution; supplying the (meth)acrylic acid aqueous solution to a crystallizer and performing crystallization to obtain purified (meth)acrylic acid and a mother liquor separated from the purified (meth)acrylic acid; supplying a part of the mother liquor to an extraction column and supplying the rest to a water separation column; bringing the mother liquor into contact with an extraction solvent in the extraction column and then supplying an upper discharge stream of the extraction column to the water separation column; performing separation in the water separation column into an upper discharge stream of the water separation column including water and a lower discharge stream of the water separation column including (meth)acrylic acid and high-boiling point by-products; and supplying the lower discharge stream of the water separation column to a high-boiling point by-product separation column and supplying an upper discharge stream of the high-boiling point by-product separation column including (meth)acrylic acid to the crystallizer.

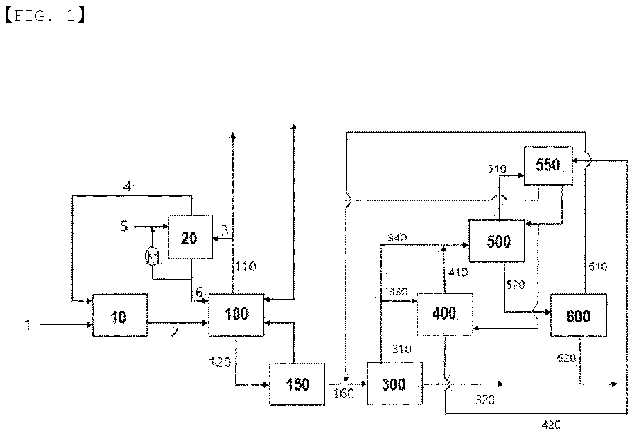

Hereinafter, each process which may be included in the exemplary embodiment of the present invention will be described, with reference to FIG. 1 and the like.

First, the method for preparation of (meth)acrylic acid according to an exemplary embodiment of the present invention may include bringing a mixed gas including (meth)acrylic acid into contact with water in an absorption column to obtain a (meth)acrylic acid aqueous solution. Herein, the mixed gas including (meth)acrylic acid is a concept which collectively refers to gas phase components discharged from a reactor 10 which produces (meth) acrylic acid by a gas phase oxidation reaction. Specifically, the mixed gas may include (meth)acrylic acid, an unreacted raw material compound, (meth)acrolein, inert gas, carbon monoxide, carbon dioxide, water vapor, various organic by-products (acetic acid, low-boiling point by-products, high-boiling point by-product, etc.), and the like. Herein, a "low-boiling point by-product" (light ends) or a "high-boiling point by-product" (heavies) is a kind of by-product which may be produced in preparation and recovery processes of the target (meth)acrylic acid, and may be a compound having a higher or lower molecular weight than the (meth)acrylic acid.

Specifically, the mixed gas including (meth)acrylic acid may be prepared as follows.

First, a reaction gas including gas containing oxygen and a raw material compound is supplied to a reactor 10 provided with a catalyst through a reaction gas supply line 1, and is subjected to a gas phase oxidation reaction in the presence of a catalyst in the reactor 10 to obtain the mixed gas including (meth)acrylic acid.

Herein, the gas containing oxygen may be air. The raw material compound may be one or more compounds selected from the group consisting of propane, propylene, butane, 1-butylene, t-butylene, and (meth)acrolein, and specifically, the raw material compound may include propylene. Meanwhile, the reaction gas supplied to the reactor 10 may further include a recirculation gas which is recovered from an upper portion of the absorption column 100 and recirculated. Therefore, the mixed gas including (meth)acrylic acid may be a reaction product by the gas phase oxidation reaction of a reactant including air, the raw material compound, and the recirculation gas, in the reactor 10.

The recirculation gas may be derived from the upper portion of the absorption column 100 described later. That is, the mixed gas comes into contact with water which is an absorption solvent in the absorption column 100, and a non-condensable gas which is not dissolved in water may be discharged as an upper discharge stream 110 of the absorption column 100. The non-condensable gas may include impurities such as acetic acid, inert gas, an unreacted raw material compound, and a minimum content of (meth)acrylic acid.

That is, when acetic acid is discharged from the upper portion of the absorption column 100, as an amount of discharged acetic acid is increased, a content of (meth)acrylic acid discharged from the upper portion of the absorption column 100 tends to be increased. This means loss of (meth)acrylic acid. As described later, according to the present invention, since acetic acid in the system may be further separated and removed by a water separation column and a layer separator after the absorption column, the acetic acid in the system does not need to be discharged forcefully to the upper discharge stream 110 of the absorption column, and specifically, the acetic acid in an amount sufficient to minimize a content of (meth)acrylic acid in the upper discharge stream 110 of the absorption column may be discharged as the upper discharge stream 110. Thus, the content of (meth)acrylic acid which is discharged from the upper portion of the absorption column 100 and lost may be minimized.

Meanwhile, a part 3 of the upper discharge stream 110 of the absorption column may be supplied to a cooling tower 20, and the rest may be supplied to a waste gas incinerator and discarded.

The cooling tower 20 is provided with a water supply line 5 in the upper portion, and water used as an absorption solvent in the absorption column may be supplied from the water supply line 5 into the cooling tower 20. In the cooling tower 20, water may come into contact with the non-condensable gas included in the part 3 of the upper discharge stream 110 of the absorption column. As described above, the non-condensable gas may include acetic acid and a minimal amount of (meth)acrylic acid, and these components are dissolved in the water, which may be discharged in an aqueous solution form as a lower discharge stream of the cooling tower 20.

Thereafter, a part 6 of the lower discharge stream of the cooling tower 20 may be supplied to the absorption column 100, and the rest may be cooled through a heat exchanger and circulated to the cooling tower.

That is, water needed in the absorption column 100 may be supplied through the water supply line 5 provided in the upper portion of the cooling tower 20. The water may include, specifically, water such as tap water and deionized water, and may include circulation process water introduced from other processes (e.g., water phase recirculated from an extraction process and/or a distillation process). Further, the absorption solvent may include a small amount of organic by-products (e.g. acetic acid) introduced from other processes.

Meanwhile, most of the acetic acid included in the non-condensable gas by a contact with water in the cooling tower 20 is removed by dissolution in water, and gas which is not dissolved in water is discharged through a recirculation gas transfer line 4 provided in the upper portion of the cooling tower 20 as a recirculation gas. The recirculation gas may be supplied to the reactor 10 so that it may be used in the gas phase oxidation reaction for preparing (meth)acrylic acid which proceeds in the reactor. The recirculation gas may be mixed with the reaction gas and supplied to the reactor, and may be supplied to the reactor through a separate line 4 from the line 1 to which the reaction gas is supplied.

Furthermore, the content of the water in the recirculation gas which is circulated from the cooling tower 20 to the reactor may be reduced by lowering a temperature inside the cooling tower 20. That is, a moisture content in a stream supplied from the reactor 10 to the absorption column 100 may be lowered by reducing the moisture content in the recirculation gas, and thus, even the moisture content in the absorption column 100 may be lowered. Various by-products which are inappropriate for being introduced to a crystallizer may be dissolved in water discharged from the reactor, and when water is present in excess in the absorption column 100, it is difficult to obtain a high-concentration (meth)acrylic acid aqueous solution, and thus, the content of water in the recirculation gas is lowered, thereby being capable of introducing a discharge stream of the absorption column 100 to the crystallizer without a separate distillation process of water, as described later.

Specifically, when the absorption solvent is water, the moisture content in the recirculation gas may be 1 wt% to 10 wt%, specifically 3 wt% to 5 wt%. When the moisture content in the recirculation gas is less than 1 wt%, operating costs, in particular, costs for equipment and operation of the cooler may be increased. Meanwhile, when the moisture content in the recirculation gas is more than 10 wt%, the content of moisture which is supplied into the absorption column 100 via the reactor 10 is increased so that high-purity (meth)acrylic acid may not be obtained and it may be difficult to simply obtain high-purity (meth)acrylic acid through the crystallizer without a process of distilling the (meth)acrylic acid aqueous solution discharged from the absorption column with water. In addition, due to the amount of moisture (water) increased in a subsequent process, energy usage required to separate and distill water may be increased.

An upper temperature of the cooling tower 20 for this may be 35°C to 55°C, specifically 35°C to 45°C. When the upper temperature of the cooling tower 20 is at least lower than 35°C, an excessive refrigerant is consumed as compared with a reduction effect of the moisture content included in the recirculation gas or a refrigerant at a lower temperature is needed, and thus, there may be no great benefit in terms of efficient energy use. Meanwhile, when the upper temperature of the cooling tower 20 is higher than 55°C, a content of the absorption solvent (moisture) included in the recirculation gas transfer line 4 is excessively increased, and thus, it may be difficult to obtain a high-concentration (meth)acrylic acid solution discharged from the absorption column 100. The temperature of the upper portion of the cooling tower 20 is controlled by a heat exchanger provided in the lower portion of the cooling tower 20, and specifically, may be controlled by circulating a part of the lower stream of the cooling tower 20 to the heat exchanger and the cooling tower 20. Meanwhile, the upper portion of the cooling tower 20 may be operated under normal pressure operation conditions.

Thereafter, a process for obtaining the (meth)acrylic acid aqueous solution by supplying the mixed gas including (meth)acrylic acid to the absorption column 100 through a reactor discharge line 2 and bringing the gas into contact with water in the absorption column 100 may be performed. Specifically, the mixed gas including (meth)acrylic acid, organic by-products, and water vapor which are produced by a synthesis reaction of (meth)acrylic acid may be brought into contact with water which is the absorption solvent in the absorption column 100 to obtain the (meth)acrylic acid aqueous solution.

Herein, the kind of absorption column 100 may be determined considering contact efficiency of the mixed gas and the absorption solvent and the like, and for example, may be a packed column type absorption column or a multistage tray type absorption column. To the inside of the packed column type absorption column, a filler such as a Rasching ring, a Pall ring, a saddle, a gauze, and a structured packing may be applied.

Further, considering the efficiency of the absorption process, the mixed gas 2 may be supplied to the lower portion of the absorption column 100, and water which is the absorption solvent may be supplied to the upper portion of the absorption column 100.

Meanwhile, the absorption column 100 may be operated at an internal pressure of 1 to 1.5 bar or 1 to 1.3 bar and at an internal temperature of 50 to 120°C or 50 to 100°C, considering the condensation conditions of (meth)acrylic acid, a moisture content depending on a saturated water vapor pressure, and the like.

Meanwhile, according to an exemplary embodiment of the present invention, the (meth)acrylic acid aqueous solution is obtained by an absorption process performed in the absorption column 100, and the (meth)acrylic acid aqueous solution may be discharged as the lower discharge stream 120 of the absorption column 100.

Meanwhile, the upper discharge stream of the absorption column 100 may include a non-condensable gas which is not dissolved in water which is the absorption solvent in the absorption column 100, as described above, and the non-condensable gas may include acetic acid, inert gas, an unreacted raw material compound, and a minimal content of (meth)acrylic acid.

Conventionally, concentration of acetic acid in the system may be prevented or a subsequent process after the absorption column may be simplified by discharging acetic acid as much as possible to the upper discharge stream of the absorption column 100. However, when the amount of acetic acid discharged to the upper portion of the absorption column 100 is increased and exceeds a certain level, the content of (meth) acrylic acid discharged to the upper portion of the absorption column 100 is increased, and thus, the amount of (meth)acrylic acid lost in the absorption column is also increased. Therefore, the present invention may minimize the content of (meth) acrylic acid lost in the absorption column, by controlling the amount of acetic acid included in the upper discharge stream of the absorption column 100. That is, the content of acetic acid in the upper discharge stream of the absorption column may be controlled so that the content of (meth)acrylic acid lost to the upper portion of the absorption column may be minimized. In addition, since acetic acid may be further separated and removed by a water separation column and a layer separator after the absorption column described later, a problem that acetic acid accumulates in the system and acts as an impurity may be solved.

From this point of view, a flow rate ratio of acetic acid discharged to the upper portion of the absorption column may be 20 wt% to 80 wt%, specifically 30 wt% to 60 wt%, based on a flow rate of the acetic acid introduced to the absorption column. Meanwhile, the content of (meth)acrylic acid included in the upper discharge stream of the absorption column may be 0.1 wt% to 0.5 wt%, specifically 0.2 wt% to 0.3 wt%.

Meanwhile, the method for preparation of (meth)acrylic acid according to an exemplary embodiment of the present invention may include supplying the (meth)acrylic acid aqueous solution to a crystallizer 300, and performing crystallization to obtain purified (meth)acrylic acid and a mother liquor separated from the purified (meth)acrylic acid.

Specifically, the (meth)acrylic acid aqueous solution may be supplied to the crystallizer 300 through an absorption column discharge line 120 as the lower discharge stream of the absorption column 100.

Meanwhile, the (meth)acrylic acid aqueous solution may be directly supplied to the crystallizer 300, but specifically, the (meth)acrylic acid aqueous solution may be supplied to a degassing column 150 before introducing the (meth)acrylic acid aqueous solution to the crystallizer, thereby removing low-boiling point by-products including acrolein, and then may be supplied to the crystallizer 300.

The acrolein may be a raw material for preparing (meth)acrylic acid and may occur as a by-product during a gas phase oxidation reaction for preparing (meth)acrylic acid. It may be preferred to remove and separate low-boiling point by-products such as acrolein before crystallization in order to obtain high-purity purified (meth)acrylic acid in the crystallizer 300. In the degassing column 150, a gas phase fraction including the low-boiling point by-products may be circulated to the absorption column 100, and the (meth)acrylic acid aqueous solution in which the low-boiling point by-products have been degassed may be introduced to the crystallizer 300 as a lower discharge stream 160 of the degassing column 150.

In this case, the content of (meth)acrylic acid in the lower discharge stream 160 of the degassing column is 85 wt% to 99 wt%, specifically 85 wt% to 95 wt%. This is a higher level than the content of the (meth) acrylic acid in the (meth)acrylic acid aqueous solution discharged from the conventional absorption column. In particular, by setting the content of the (meth)acrylic acid in the (meth)acrylic acid aqueous solution to 85 wt% or more, the (meth)acrylic acid aqueous solution may be directly supplied to the crystallizer 300 without undergoing a separate purification process or separation process for the (meth)acrylic acid aqueous solution, and thus, overall process energy may be reduced, and also, high-purity (meth)acrylic acid may be obtained in the crystallizer 300.

The (meth)acrylic acid aqueous solution having a high (meth)acrylic acid content as such may be achieved by, for example, optimally controlling the operation conditions of the cooling tower 20 and the absorption column 100 depending on the material component in the system and its content to minimize the moisture content in the absorption column 100. That is, an absorption solvent component in the recirculation gas which circulates from the cooling tower 20 to the reactor 10 is minimized, and the addition amount and the usage amount of water supplied to the cooling tower 20 and the absorption column 100 are minimized, thereby implementing the (meth)acrylic acid aqueous solution having a high (meth)acrylic acid concentration.

Meanwhile, the (meth)acrylic acid included in the (meth)acrylic acid aqueous solution supplied to the crystallizer 300 may be recrystallized through a crystallization process and obtained as crystallized high-purity (meth)acrylic acid. The (meth)acrylic acid which is crystallized in the crystallizer may be referred to as purified (meth)acrylic acid in some cases, in the present specification. The crystallization process may be performed under common conditions.

The crystallization method for obtaining a product by crystallization in the present invention may be suspension crystallization and layer crystallization without limitation, may be continuous or batchwise, and may be performed in one stage or two stages or more. As a non-limited example, the (meth)acrylic acid may be dynamically crystallized and provided as purified (meth)acrylic acid.

Specifically, in order to dynamically crystallize the (meth)acrylic acid before crystallization, the (meth)acrylic acid aqueous solution may be first allowed to flow on a tube inner wall in a falling film form. Further, the temperature of the tube may be adjusted to a freezing point of (meth)acrylic acid or lower to form a crystal in the tube inner wall. Then, the temperature of the tube may be raised to near the freezing point of (meth) acrylic acid to sweat about 5 wt% of (meth)acrylic acid. Further, the mother liquor sweated from the tube is removed and the crystal formed in the tube inner wall is recovered, thereby obtaining the high-purity purified (meth)acrylic acid. The mother liquor may refer to a solution which is the (meth)acrylic acid aqueous solution introduced to the crystallizer 300 from which the purified (meth)acrylic acid has been removed. Therefore, the mother liquor may include (meth)acrylic acid, water, acetic acid, and high-boiling point by-products, in which the (meth)acrylic acid is residual (meth)acrylic acid which is not crystallized in the crystallizer 300, and may be separated in a high-boiling point by-product separation column 600 described later and circulated to the crystallizer 300 again.

Separation of the mother liquor and the crystallized (meth)acrylic acid may be performed using a solid-liquid separation device, for example, a belt filter, a centrifuge, and the like. The purified (meth) acrylic acid may be recovered as a (meth)acrylic acid recovery stream 320, the mother liquor is discharged from the crystallizer 300 through a mother liquor recovery line 310, and a part of the discharged mother liquor is supplied to an extraction column 400 and the rest may be supplied to a water separation column 500.

Specifically, the mother liquor recovery line 310 branches into a first discharge line 330 of the crystallizer and a second discharge line 340 of the crystallizer, and the first discharge line 330 of the crystallizer may be connected to an extraction column 400 and the second discharge line 340 of the crystallizer may be connected to the water separation column 500. The mother liquor is discharged from the crystallizer 300 through the mother liquor recovery line 310, and then may be divided and supplied to the extraction column 400 and the water separation column 500, respectively.

Meanwhile, the mother liquor discharged from the crystallizer 300 may include 50 wt% to 80 wt%, specifically 60 wt% to 70 wt% (meth) acrylic acid. In addition, the mother liquor may include 20 wt% to 50 wt%, specifically 30 wt% to 40 wt% water.

According to the present invention, by introducing the extraction column 400 before the water separation column 500, processing burden and energy consumption of the (meth)acrylic acid aqueous solution in the water separation column 500 may be greatly decreased. Furthermore, in the method according to the present invention, the (meth)acrylic acid aqueous solution obtained from the absorption column 100 is divided and supplied to the extraction column 400 and the water separation column 500, thereby reducing an amount of water to be distilled in the water separation column 500 through extraction to reduce energy usage, while reducing overall facility burden.

A ratio of dividing and supplying the mother liquor to the extraction column 400 and the water separation column 500 may be determined considering a capacity ratio of the extraction column 400 and the water separation column 500, processing ability, an effect of energy efficiency increase of the entire process, and the like. Considering the above conditions, a flow rate of the mother liquor supplied to the extraction column 400 with respect to the entire flow rate of the mother liquor discharged through the mother liquor recovery line 310 may be 20 wt% to 60 wt%, specifically 30 wt% to 50 wt%, and in this case, is advantageous in terms of expressing the above-described effect. The rest other than the flow rate supplied to the extraction column 400 may be supplied to the water separation column 500 as described above.

Meanwhile, as the amount of the mother liquor supplied to the extraction column 400 is increased, the processing burden efficiency of the water separation column 500 may be improved to improve energy efficiency of the entire process. However, when a more than necessary amount of the mother liquor is supplied to the extraction column 400, the extraction column 400 having a larger capacity is required, and operation conditions of the water separation column 500 in the rear stage become worse to rather decrease process efficiency such as increasing loss of (meth)acrylic acid, and thus, it is advantageous that a supply ratio of the mother liquor is adjusted within the range described above. In addition, as the amount of the mother liquor supplied to the water separation column 500 is larger, the amount of water to be removed by azeotropic distillation in the water separation column 500 is increased, so that the energy use reduction effect according to the present invention may be decreased, and thus, it is advantageous that a supply ratio of the mother liquor is adjusted within the range described above.

According to an exemplary embodiment of the present invention, an extraction process step of bringing the extraction solvent and the mother liquor into contact in the extraction column 400 to obtain an extract and a raffinate, and supplying an extract stream 410 including the extract to the water separation column 500 may be included.

The extraction column 400 is supplied with a part of the mother liquor discharged from the crystallizer 300, removes most of the water included in the mother liquor without using much energy, and supplies it to the water separation column 500, thereby saving energy used in azeotropic distillation in the water separation column 500 described later. In this respect, it is preferred that extraction in the extraction column 400 uses a liquid-liquid contact method in terms of improving energy efficiency of the entire process.

Herein, the extraction solvent may be a hydrocarbon solvent which forms an azeotrope with water and an organic by-products (acetic acid, etc.) and does not form an azeotrope with (meth)acrylic acid but may sufficiently extract (meth)acrylic acid, and also, it is advantageous in the extraction process that its boiling point is 10 to 120°C. Specifically, the extraction solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

Meanwhile, it is advantageous in the process that a temperature of the mother liquor in the extraction process is 10 to 70°C. Further, it is advantageous in the process that a weight ratio of the extraction solvent to the mother liquor in the extraction process is 1:1 to 1:5, preferably 1:1.2 to 1:2.5.

In addition, as the extraction column 400, an extraction device according to the liquid-liquid contact method may be used. A non-limiting example of the extraction device may include a Karr reciprocating plate column, a rotary-disk contactor, a Scheibel column, a Kuhni column, a spray extraction column, a packed extraction column, a pulsed packed column, a bank of mixer-settler, mixer and centrifuge (centrifugal counter current extractor), and the like.

In this way, a significant portion of water in the mother liquor supplied to the extraction column 400 through the first discharge line 330 of the crystallizer is removed, an extract from which (meth)acrylic acid is extracted by an extraction solvent is obtained, and the extract may be supplied to the water separation column 500 as an extract stream 410. Specifically, the extract may include (meth)acrylic acid, acetic acid, the extract solvent, and high-boiling point by-products.

In addition, water included in the mother liquor may be recovered as a raffinate through the extraction process. The recovered raffinate may be discharged as a raffinate stream 420 and introduced to a layer separator 550 described later. Since water is recovered in the extraction process as such, the operation burden of the distillation process described later may be decreased to greatly lower energy consumption.

According to an exemplary embodiment of the present invention, the stream supplied to the water separation column 500 may include the mother liquor supplied along the second discharge line 340 of the crystallizer and the extract supplied along the extract stream 410.

A flow rate of water in the stream supplied to the water separation column 500 may be 20 wt% to 50 wt%, specifically 25 wt% to 40 wt%, based on the flow rate of the water introduced to the absorption column 100.

A distillation process in the water separation column 500 for the stream supplied to the water separation column 500 may be a process of separating an upper fraction including water and acetic acid and a lower fraction including (meth)acrylic acid and high-boiling point by-products by azeotropic distillation.

According to the present invention, distillation in the water separation column 500 is performed in the presence of a hydrophobic azeotropic solvent, and it is advantageous in the process since the hydrophobic azeotropic solvent, water, and organic by-products (such as acetic acid) may be simultaneously recovered.

Herein, the hydrophobic azeotropic solvent is a hydrophobic solvent which may form an azeotrope with water and acetic acid and does not form an azeotrope with (meth)acrylic acid, and a hydrocarbon-based solvent satisfying the physical properties may be applied without limitation. Further, the hydrophobic azeotropic solvent may have a lower boiling point than (meth)acrylic acid, and may have a boiling point of preferably 10 to 120°C.

According to the present invention, the hydrophobic azeotropic solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

Further, the hydrophobic azeotropic solvent may be the same as or different from the extraction solvent applied to the extraction column 400. However, considering production efficiency according to a continuous process and the like, it is preferred that the hydrophobic azeotropic solvent is the same as the extraction solvent. When the same compound is used as the hydrophobic azeotropic solvent and the extraction solvent as such, at least a part of the hydrophobic azeotropic solvent which is distilled in the water separation column 500 and recovered may be supplied to a lower portion of the extraction column 400 and used as a part of the extraction solvent.

When the hydrophobic azeotropic solvent is added to the water separation column 500 described above, an azeotrope of (meth)acrylic acid and water is broken. Accordingly, water, acetic acid, and the hydrophobic azeotropic solvent used in azeotropic distillation in the mother liquor may form an azeotrope together and be recovered as an upper fraction of the water separation column 500. Further, a lower fraction including (meth)acrylic acid and high-boiling point by-products may be recovered to a lower portion of the water separation column 500.

The upper fraction of the water separation column recovered as such may be supplied to a layer separator 550 through an upper discharge stream 510 of the water separation column, and a lower fraction of the water separation column may be supplied to the high-boiling point by-product separation column 600 through a lower discharge stream 520 of the water separation column.

Herein, the layer separator 550 is a liquid-liquid layer separator and a device for separating fluids which are not mixed with each other by a density difference using gravity, centrifugal force, or the like, in which a relatively light liquid may be separated to the upper portion of the layer separator 550 and a relatively heavy liquid may be separated to the lower portion of the layer separator 550. Specifically, the upper discharge stream 510 of the water separation column supplied to the layer separator 550 may be separated into an organic layer including a hydrophobic azeotropic solvent and a water layer including water and acetic acid.

In addition, at least a part of the organic layer separated in the layer separator 550 may be supplied to the upper portion of the water separation column 500 and reused as the hydrophobic azeotropic solvent and the rest may be supplied to the extraction column 400 and reused as the extraction solvent.

Meanwhile, at least a part of the water layer separated in the layer separator 550 may be supplied to the upper portion of the absorption column 100 and used as an absorption solvent, and the rest may be discharged as waste water.

Herein, acetic acid may be included in the water layer, and a concentration of acetic acid included in the water layer may vary depending on the kind of hydrophobic azeotropic solvent, a reflux ratio of a column installed in the water separation column 500, and the like. According to the present invention, the concentration of acetic acid included in the water layer may be 1 to 30 wt%, preferably 2 to 20 wt%, and more preferably 3 to 10 wt%.

That is, according to an exemplary embodiment of the present invention, acetic acid may be discharged through the upper discharge stream of the absorption column 100, and also discharged by azeotropic distillation performed through the water separation column 500 and the layer separator 550. Therefore, acetic acid accumulating in the system may be more efficiently removed in this process than in a process of removing acetic acid only in the upper portion of the absorption column, thereby minimizing a loss amount of (meth)acrylic acid. Furthermore, process flexibility may be secured in this process as compared with the case of attempting to discharge the entire amount of acetic acid in the system to the upper portion of the absorption column.

From this point of view, a total flow rate of acetic acid introduced to the absorption column 100 may be the same as the sum of a flow rate of acetic acid in the upper discharge stream of the absorption column 100 and a flow rate of acetic acid in a stream which is included water layer of the layer separator 550 and is discharged.

Meanwhile, the method for preparation of (meth)acrylic acid according to an exemplary embodiment of the present invention may include supplying the lower discharge stream of the water separation column 500 to the high-boiling point by-product separation column 600 and supplying the upper discharge stream of the high-boiling point by-product separation column 600 including (meth)acrylic acid to the crystallizer 300.

The lower discharge stream of the water separation column 500 may be distilled by the high-boiling point by-product separation column 600 to be separated into a lower fraction including high-boiling point by-products and an upper fraction including a high content of (meth)acrylic acid by removing the high-boiling point by-products. The upper fraction may be supplied to the crystallizer 300 through the upper discharge stream 610 of the high-boiling point by-product separation column, and the content of (meth)acrylic acid included in the upper discharge stream 510 of the high-boiling point by-product separation column may be 90 wt% to 99 wt%, specifically 95 wt% to 99 wt%.

That is, since the amount water included in each stream is already been reduced in the process after the absorption column 100 and also a significantly amount water is removed through the extraction column 400 and the water separation column 500, the content of water in the upper discharge stream 610 of the high-boiling point by-product separation column is controlled to be low, and thus, a highly concentrated stream of (meth)acrylic acid which may be directly introduced to the crystallizer 300 may be implemented. The loss of (meth) acrylic acid may be decreased to the maximum by introducing the upper discharge stream 610 of the high-boiling point by-product separation column to the crystallizer 300.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Example 1

According to the process flow illustrated in FIG. 1, a preparation process of (meth)acrylic acid was simulated, using an Aspen Plus simulator available from Aspen.

Specifically, a reaction gas including air and a raw material compound (propylene) was supplied to a reactor 10 provided with a catalyst through a reaction gas supply line 1, and a recirculation gas derived from a cooling tower 20 was supplied to the reactor 10 through a recirculation gas transfer line 4. A mixed gas 2 having a composition including (meth)acrylic acid (7.0 mol%), water (11.8 mol%), a high-boiling point material (0.09 mol%), and inert gas (80.6 mol%) was obtained by a gas phase oxidation reaction performed in the reactor 10.

The mixed gas 2 was added to a 22nd stage from the top of an absorption column 100 at a temperature of 164°C. In the absorption column 100, the mixed gas was brought into contact with an absorption solvent (water) to obtain a (meth)acrylic acid aqueous solution. The water added to the absorption column 100 was supplied through a water layer from a lower discharge stream 6 of the cooling tower and a water layer from a layer separator 550 described later, and the water was supplied to the upper portion of the absorption column 100 at a mass flow rate of 5.8 wt% with respect to the flow rate of the mixed gas 2. A mass flow rate ratio of acetic acid discharged out of the system through the upper discharge stream 110 of the absorption column was 40.8 wt%, based on the flow rate of the acetic acid introduced to the absorption column 100. At this time, the pressure of the upper portion of the absorption column 100 was 1.1 bar, and a temperature of the lower portion of the absorption column 100 was 84.1°C.

In the absorption column 100, a non-condensable gas including components which were not dissolved in water was separated as the upper discharge stream 110 of the absorption column, and a part 3 of the upper discharge stream of the absorption column was supplied to the cooling tower 20 and the rest was discharged out of the system. In the cooling tower 20, the non-condensable gas included in the part 3 of the upper discharge stream of the absorption column was dissolved in water. At this time, the water was supplied through a water supply line 5. In the cooling tower 20, the gas which was not dissolved in water was supplied to the reactor 10 through a recirculation gas transfer line 4, and a part of the lower discharge stream 6 of the cooling tower including water and components dissolved in water (acetic acid and (meth)acrylic acid which was not dissolved in water in the absorption column) was supplied to the upper portion of the absorption column 100.

Meanwhile, the (meth)acrylic acid aqueous solution described above was supplied to a degassing column 150 as the lower discharge stream 120 of the absorption column and degassed, and low-boiling point by-products were supplied to the absorption column 100 as an upper discharge stream of the degassing column and a (meth)acrylic acid aqueous solution in which the low-boiling point by-products were degassed was supplied to the crystallizer 300 as a lower discharge stream 160 of the degassing column. At this time, the upper discharge stream of the degassing column was supplied to the absorption column 100 at a flow rate of 25 wt% with respect to the mass flow rate of water added to the absorption column 100. Meanwhile, the composition of the lower discharge stream 160 of the degassing column included (meth)acrylic acid (89.5 wt%), acetic acid (1.8 wt%), water (7 wt%), furfural (0.8 wt%), and maleic acid (0.8 wt%).

The lower discharge stream 160 of the degassing column was mixed with an upper discharge stream 610 of the high-boiling point by-product separation column described later and supplied to the crystallizer 300, and the mixed stream included (meth)acrylic acid (90.9 wt%), acetic acid (1.6 wt%), water (5.9 wt%), furfural (0.8 wt%), and maleic acid (0.7 wt%).

A (meth)acrylic acid recovery stream 320 including (meth)acrylic acid was obtained by a crystallization process performed in the crystallizer 300, thereby obtaining (meth)acrylic acid. The content of the (meth)acrylic acid in the (meth)acrylic acid recovery stream 320 was 99.5 wt% or more.

In addition, a mother liquor separated from the (meth)acrylic acid was obtained in the crystallizer 300. At this time, the mother liquor included (meth)acrylic acid (64 wt%), water (23.7 wt%), acetic acid (6.5 wt%), and high-boiling point by-products (6.0 wt%).

The mother liquor discharged through a mother liquor recovery line 310 branched, and 40 wt% of the mother liquor with respect to the total weight of the mother liquor was supplied to the extraction column 400 through a first discharge line 330 of the crystallizer and the rest of the mother liquor of 60 wt% was supplied to a water separation column 500 through a second discharge line 340 of the crystallizer. Meanwhile, the second discharge line 340 of the crystallizer was mixed with an extract stream 410 described later and supplied to the water separation column 500.

A raffinate stream 420 including water and the extract stream 410 including toluene and (meth)acrylic acid were obtained, by an extraction process performed in the presence of an extraction solvent (toluene) in the extraction column 400. At this time, toluene supplied to the lower portion of the extraction column 400 was supplied at a mass flow rate of 4.5 times the flow rate of water in the mother liquor supplied to the extraction column 400. The raffinate stream 420 was supplied to a layer separator 550 described later, and the extract stream 410 was supplied to the water separation column 500.

In the water separation column 500, an upper discharge stream 510 of the water separation column including toluene, water, and acetic acid and a lower discharge stream 520 of the water separation column including (meth)acrylic acid and high-boiling point by-products were obtained, by azeotropic distillation performed in the presence of a hydrophobic azeotropic solvent (toluene). At this time, toluene supplied to the water separation column 500 was supplied at a mass flow rate twice the flow rate of toluene supplied to the extraction column 400.

The upper discharge stream 510 of the water separation column was supplied to a layer separator 550 to be separated into a water layer including water and acetic acid and an organic layer including toluene. A part of the water layer was supplied to the absorption column 100 and the rest was discharged out of the system as waste water. Meanwhile, the organic layer was supplied to the extraction column 400 and the water separation column 500.

Meanwhile, the lower discharge stream 520 of the water separation column described above was supplied to a high-boiling point by-product separation column 600 to obtain an upper discharge stream 610 of the high-boiling point by-product separation column including (meth)acrylic acid and a lower discharge stream 620 of the high-boiling point by-product separation column including high-boiling point by-products. The upper discharge stream 610 of the high-boiling point by-product separation column was mixed with a lower discharge stream 160 of the degassing column and supplied to the crystallizer 300, as described above. At this time, the content of (meth)acrylic acid in the upper discharge stream 610 of the high-boiling point by-product separation column was 98.6 wt%.

As a result, energy used in the water separation column 500 was 89.1 kcal/kg AA and energy used in the high-boiling point by-product separation column 600 was 35.9 kcal/kg AA, and thus, a total of 125 kcal/kg AA of energy was used.

Meanwhile, the amount of (meth)acrylic acid lost through the upper discharge stream 110 of the absorption column was 0.92 wt% with respect to the production amount of (meth)acrylic acid (amount of (meth)acrylic acid obtained from the crystallizer), and the amount of (meth)acrylic acid lost through waste water derived from the layer separator 250 was 0.13 wt% with respect to the production amount of (meth)acrylic acid, and thus, a total of 1.05 wt% of (meth)acrylic acid loss occurred.

### Comparative Example 1

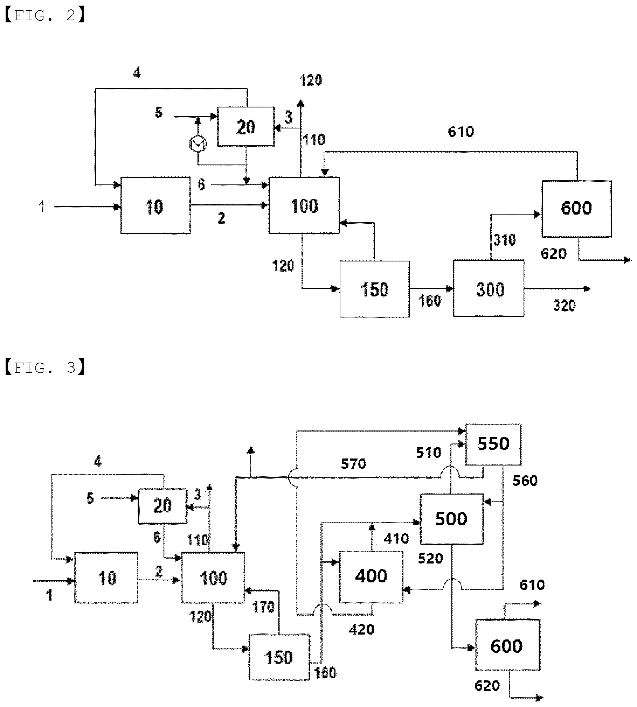

According to the process flow illustrated in FIG. 2, a preparation process of (meth)acrylic acid was simulated, using an Aspen Plus simulator available from Aspen.

Specifically, in Comparative Example 1, a (meth)acrylic acid solution was obtained from the bottom of the absorption column 100, and the (meth)acrylic acid solution was added to a crystallizer 300 through a degassing column 150 to obtain (meth)acrylic acid 320. At this time, water added to the absorption column 100 was supplied as a mixture of a stream 6 directly supplied to the absorption column and a part of a lower discharge stream of a cooling tower, and the water was supplied to an upper portion of the absorption column at a mass flow rate of 6.6 wt% with respect to the flow rate of the mixed gas 2.

In the crystallizer 300, the mother liquor 310 after crystallization was added to the high-boiling point by-product separation column 600 to obtain a lower discharge stream 620 of the high-boiling point by-product separation column including the high-boiling point by-products and an upper discharge stream 610 of the high-boiling point by-product separation column including the mother liquor from which the high-boiling point by-products had been removed. The upper discharge stream 610 of the high-boiling point by-product separation column was recirculated to the absorption column 100. The (meth)acrylic acid was prepared in the same manner as in Example 1, except the above.

A lower discharge stream 160 of the degassing column added to the crystallizer 300 included (meth)acrylic acid (90.5 wt%), acetic acid (1.8 wt%), water (5.9 wt%), furfural (0.7 wt%), and maleic acid (0.7 wt%). As a result, 99.5 wt% or more of (meth)acrylic acid was obtained from the (meth)acrylic acid recovery stream 320 of the crystallizer 300.

Energy used for removing high-boiling point by-products from the mother liquor after crystallization in the high-boiling point by-product separation column 600 was 154.5 kcal/kg AA. Meanwhile, the amount of (meth)acrylic acid lost through the upper discharge stream 110 of the absorption column was 1.56 wt% with respect to the production amount of (meth)acrylic acid.

Comparative Example 1 is the case in which the mother liquor 310 was supplied to the high-boiling point by-product separation column 600, and total energy usage was increased and the loss of (meth) acrylic acid was also increased by about 1.5 times, as compared with Example 1.

### Comparative Example 2

According to the process flow illustrated in FIG. 3, a preparation process of (meth)acrylic acid was simulated, using an Aspen Plus simulator available from Aspen.

Specifically, in Comparative Example 1, the lower discharge stream 160 of the degassing column having the same composition as Example 1 was not supplied to the crystallizer, and the lower discharge stream 160 of the degassing column branched to supply 40 wt% of the total weight of the lower discharge stream 160 of the degassing column to the extraction column 400 and the rest of 60 wt% to the water separation column 500.

However, since the content of (meth)acrylic acid in the branched lower discharge stream of the degassing column supplied to the extraction column 400 was very high, the extraction process was not performed even with the supply of the extraction solvent to the extraction column 400, and thus, it was confirmed that phase separation did not occur. Furthermore, it was impossible to finally obtain (meth)acrylic acid.

## Claims

1. A method for preparation of (meth)acrylic acid, the method comprising:
bringing a mixed gas including (meth)acrylic acid into contact with water in an absorption column to obtain a (meth)acrylic acid aqueous solution;
supplying the (meth)acrylic acid aqueous solution to a crystallizer and performing crystallization to obtain purified (meth)acrylic acid and a mother liquor separated from the purified (meth)acrylic acid;
supplying a part of the mother liquor to an extraction column and supplying the rest to a water separation column;
bringing the mother liquor into contact with an extraction solvent in the extraction column to obtain a raffinate and an extract, and then supplying an extract stream including the extract to the water separation column;
performing separation in the water separation column into an upper discharge stream of the water separation column including water and a lower discharge stream of the water separation column including (meth)acrylic acid and high-boiling point by-products; and
supplying the lower discharge stream of the water separation column to a high-boiling point by-product separation column and supplying an upper discharge stream of the high-boiling point by-product separation column including (meth)acrylic acid to the crystallizer.

2. The method for preparation of (meth)acrylic acid of claim 1, wherein the (meth)acrylic acid aqueous solution is supplied to a degassing column, and a lower discharge stream of the degassing column from which low-boiling point by-products have been removed is supplied to the crystallizer.

3. The method for preparation of (meth)acrylic acid of claim 2, wherein a content of (meth)acrylic acid in the lower discharge stream of the degassing column is 85 wt% to 99 wt%.

4. The method for preparation of (meth)acrylic acid of claim 1, wherein the mother liquor includes (meth)acrylic acid, water, acetic acid, and high-boiling point by-products.

5. The method for preparation of (meth)acrylic acid of claim 1, wherein the upper discharge stream of the water separation column is supplied to a layer separator, a part of a water phase including water and acetic acid is circulated to the absorption column, and the rest is discharged as waste water.

6. The method for preparation of (meth)acrylic acid of claim 1, wherein the raffinate includes water, and a raffinate stream including the raffinate is supplied to the layer separator.

7. The method for preparation of (meth)acrylic acid of claim 1, wherein a content of the (meth)acrylic acid in the upper discharge stream of the high-boiling point by-product separation column is 95 wt% to 99 wt%.

8. The method for preparation of (meth)acrylic acid of claim 1, wherein a flow rate ratio of acetic acid discharged to an upper portion of the absorption column is 30 wt% to 60 wt%, based on a flow rate of the acetic acid introduced to the absorption column.

9. The method for preparation of (meth)acrylic acid of claim 1, wherein the mother liquor includes 50 wt% to 80 wt% (meth)acrylic acid and 20 wt% to 50 wt% water.

10. The method for preparation of (meth)acrylic acid of claim 1,
wherein the mixed gas including (meth)acrylic acid is a reaction product by a gas phase oxidation reaction of a reactant including air, a raw material compound, and a recirculation gas in the reactor, and
the recirculation gas is that, after a part of the upper discharge stream of the absorption column is supplied to the cooling tower and cooled, discharged as an upper discharge stream of the cooling tower and circulated to the reactor.
